# EUROPEAN PATENT APPLICATION

(11) **EP 1 480 488 A1**
(43) Date of publication of application: **24.11.2004**
(21) Application number: 04252611.1
(22) Date of filing: 05.05.2004
(51) Int. Cl.: H04Q 7/38

(54) **Controlled time scheduling**

(30) Priority: 21.05.2003 US 442785
(71) Applicant: LUCENT TECHNOLOGIES INC., Murray Hill, New Jersey 07974-0636 (US)
(72) Inventor: Liu, Jung-Tao, New Jersey, 07940 (US)
(74) Representative: Watts, Christopher Malcolm Kelway, Dr.

(57) **Abstract**

A method for controlling the flow of information between a UE (120) and a Node B (130) is provided. The UE (120) transmits over a control channel (240,280) to the Node B (130) a signal requesting to transmit information to the Node B (130). The UE (120) then receives over a shared channel (220,260) from the Node B (130) a signal identifying a time at which the UE (120) is permitted to transmit information. Thereafter, the UE (120) transmits at the identified time over a data channel (230,270) to the Node B (130) a signal containing the information.

## Description

### BACKGROUND OF THE INVENTION

### 1. FIELD OF THE INVENTION

This invention relates generally to telecommunications, and, more particularly, to wireless communications.

### 2. DESCRIPTION OF THE RELATED ART

In the field of wireless telecommunications, such as cellular telephony, a system typically includes a plurality of Node Bs (*e.g.*, base stations) distributed within an area to be serviced by the system. Various users within the area, fixed or mobile, may then access the system and, thus, other interconnected telecommunications systems, via one or more of the Node Bs. Typically, a UE (e.g., a user) maintains communications with the system as the user passes through an area by communicating with one and then another Node B, as the user moves. The user may communicate with the closest Node B, the Node B with the strongest signal, the Node B with a capacity sufficient to accept communications, *etc.*

Commonly, each Node B is constructed to process a plurality of communications sessions with a plurality of users in parallel. In this way, the number of Node Bs may be limited while still providing communications capabilities to a large number of simultaneous users. Typically, each user is generally free to transmit information to the Node B without regard to the status of other users. That is, multiple users may transmit information to the Node B at the same time. This unregulated transfer of information, however, may result in interference between users whose transmissions overlap, even partially.

In systems that transmit voice, or even data at relatively low speeds, the interference caused by overlapping transmissions does not significantly impact the quality of the communications session. However, as use of the Internet, e-mail and other data-intensive services have become ubiquitous, wireless communications systems are now attempting to provide some of these same services. These types of services, however, require large amounts of data to be transmitted at relatively high speed. In a system that is intended to transmit large amounts of data at high speed, the interference can be significant. In fact, the interference caused by overlapping transmissions can impose substantial limits on the speed at which data can be transmitted, rendering high-speed data communications unworkable in some instances.

The present invention is directed to overcoming, or at least reducing, the effects of one or more of the problems set forth above.

### SUMMARY OF THE INVENTION

In one aspect of the instant invention, a method is provided for controlling a flow of information in a communications system. The method may comprise transmitting a signal requesting to transmit information. A signal identifying a time at which information is permitted to be transmitted may be received. Thereafter, a signal containing the information may be transmitted at the identified time.

In another aspect of the instant invention, a device for controlling a flow of information is provided. The device may comprise a control channel, a data channel, a shared channel, and a controller. The controller is adapted to transmit over the control channel a signal requesting to transmit information from the device, to receive over the shared channel a signal identifying a time at which the device is permitted to transmit information, and to transmit at the identified time over the data channel a signal containing the information.

In yet another aspect of the instant invention, a system is provided. The system may comprise a Node B and a UE. The UE may be adapted to transmit a signal requesting to transmit information from the UE to the Node B. The UE may also receive a signal identifying a time at which the UE may be permitted to transmit information, and may transmit at the identified time to the Node B a signal containing the information.

In still another aspect of the instant invention, a method for controlling a flow of information is provided. The method may comprise receiving a signal requesting to transmit information. A signal identifying a time at which information is permitted to be transmitted may then be transmitted. At the identified time a signal containing the information may be received.

### BRIEF DESCRIPTION OF THE DRAWINGS

The invention may be understood by reference to the following description taken in conjunction with the accompanying drawings, in which like reference numerals identify like elements, and in which:
Figure 1 is a block diagram of a communications system, in accordance with one embodiment of the present invention;
Figure 2 depicts a block diagram of one embodiment of a Node B and a UE used in the communications system of Figure 1;
Figure 3 is a flow diagram illustrating the interoperation of the Node B and the UE of Figures 1 and 2; and
Figure 4 is a timing diagram illustrating the interoperation of the Node B and two UEs of Figures 1 and 2.

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the spirit and scope of the invention as defined by the appended claims.

### DETAILED DESCRIPTION OF SPECIFIC EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

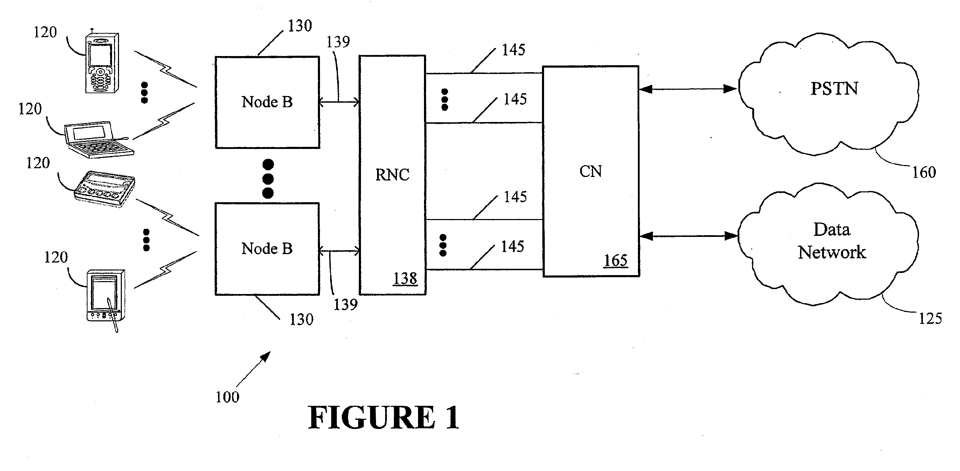

Turning now to the drawings, and specifically referring to Figure 1, a communications system 100 is illustrated, in accordance with one embodiment of the present invention. For illustrative purposes, the communications system 100 of Figure 1 is a Universal Mobile Telephone System (UMTS), although it should be understood that the present invention may be applicable to other systems that support data and/or voice communication. The communications system 100 allows one or more UEs 120 to communicate with a data network 125, such as the Internet, through one or more Node Bs 130. The UE 120 may take the form of any of a variety of devices, including cellular phones, personal digital assistants (PDAs), laptop computers, digital pagers, wireless cards, and any other device capable of accessing the data network 125 through the Node B 130.

In one embodiment, a plurality of the Node Bs 130 may be coupled to a Radio Network Controller (RNC) 138 by one or more connections 139, such as T1/EI lines or circuits, ATM circuits, cables, optical digital subscriber lines (DSLs), and the like. Although only two RNCs 138 are illustrated, those skilled in the art will appreciate that a plurality of RNCs 138 may be utilized to interface with a large number of Node Bs 130. Generally, the RNC 138 operates to control and coordinate the Node Bs 130 to which it is connected. The RNC 138 of Figure 1 generally provides replication, communications, runtime, and system management services. The RNC 138, in the illustrated embodiment handles calling processing functions, such as setting and terminating a call path and is capable of determining a data transmission rate on the forward and/or reverse link for each UE 120 and for each sector supported by each of the Node Bs 130.

The NRC 138 is, in turn, coupled to a Core Network (CN) 165 via a connection 145, which may take on any of a variety of forms, such as T1/EI lines or circuits, ATM circuits, cables, optical digital subscriber lines (DSLs), and the like. Generally the CN 140 operates as an interface to a data network 125 and/or to a public telephone system (PSTN) 160. The CN 140 performs a variety of functions and operations, such as user authentication, however, a detailed description of the structure and operation of the CN 140 is not necessary to an understanding and appreciation of the instant invention. Accordingly, to avoid unnecessarily obfuscating the instant invention, further details of the CN 140 are not presented herein.

The data network 125 may be a packet-switched data network, such as a data network according to the Internet Protocol (IP). One version of IP is described in Request for Comments (RFC) 791, entitled "Internet Protocol," dated September 1981. Other versions of IP, such as IPv6, or other connectionless, packet-switched standards may also be utilized in further embodiments. A version of IPv6 is described in RFC 2460, entitled "Internet Protocol, Version 6 (IPv6) Specification," dated December 1998. The data network 125 may also include other types of packet-based data networks in further embodiments. Examples of such other packet-based data networks include Asynchronous Transfer Mode (ATM), Frame Relay networks, and the like.

As utilized herein, a "data network" may refer to one or more communication networks, channels, links, or paths, and systems or devices (such as routers) used to route data over such networks, channels, links, or paths.

Thus, those skilled in the art will appreciate that the communications system 100 facilitates communications between the EUs 120 and the data network 125. It should be understood, however, that the configuration of the communications system 100 of Figure 1 is exemplary in nature, and that fewer or additional components may be employed in other embodiments of the communications system 100 without departing from the spirit and skill of the instant invention.

Unless specifically stated otherwise, or as is apparent from the discussion, terms such as "processing" or "computing" or "calculating" or "determining" or "displaying" or the like, refer to the action and processes of a computer system, or similar electronic computing device, that manipulates and transforms data represented as physical, electronic quantities within the computer system's registers and memories into other data similarly represented as physical quantities within the computer system's memories or registers or other such information storage, transmission or display devices.

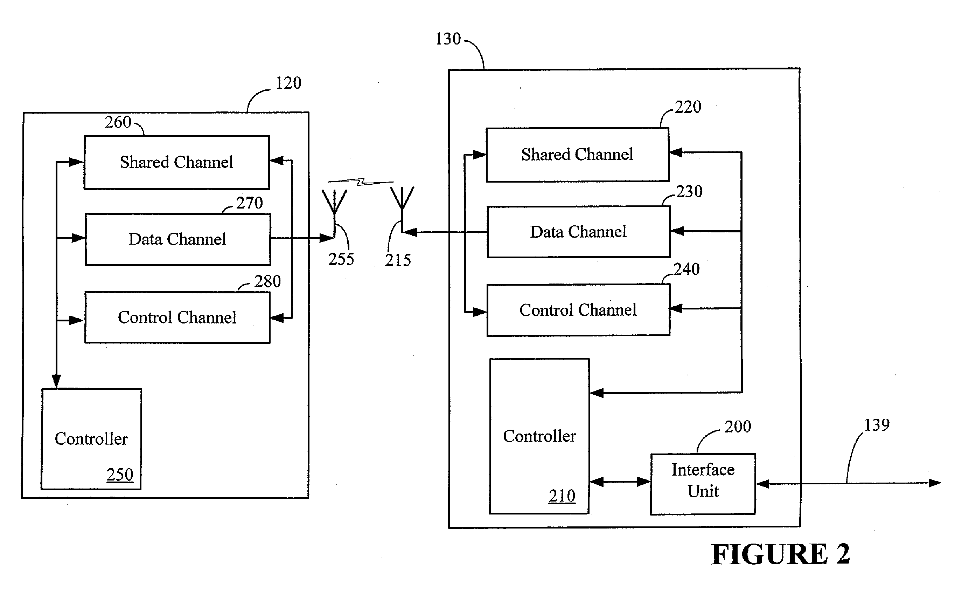

Referring now to Figure 2, a block diagram of one embodiment of a functional structure associated with an exemplary Node B 130 and UE 120 is shown. The Node B 130 includes an interface unit 200, a controller 210, an antenna 215 and a plurality of channels: a shared channel 220, a data channel 230, and a control channel 240. The interface unit 200, in the illustrated embodiment, controls the flow of information between the Node B 130 and the RNC 138 (see Figure 1). The controller 210 generally operates to control both the transmission and reception of data and control signals over the antenna 215 and the plurality of channels 220, 230, 240 and to communicate at least portions of the received information to the RNC 138 via the interface unit 200.

The UE 120 shares certain functional attributes with the Node B 130. For example, the UE 120 includes a controller 250, an antenna 255 and a plurality of channels: a shared channel 260, a data channel 270, and a control channel 280. The controller 250 generally operates to control both the transmission and reception of data and control signals over the antenna 255 and the plurality of channels 260, 270, 280.

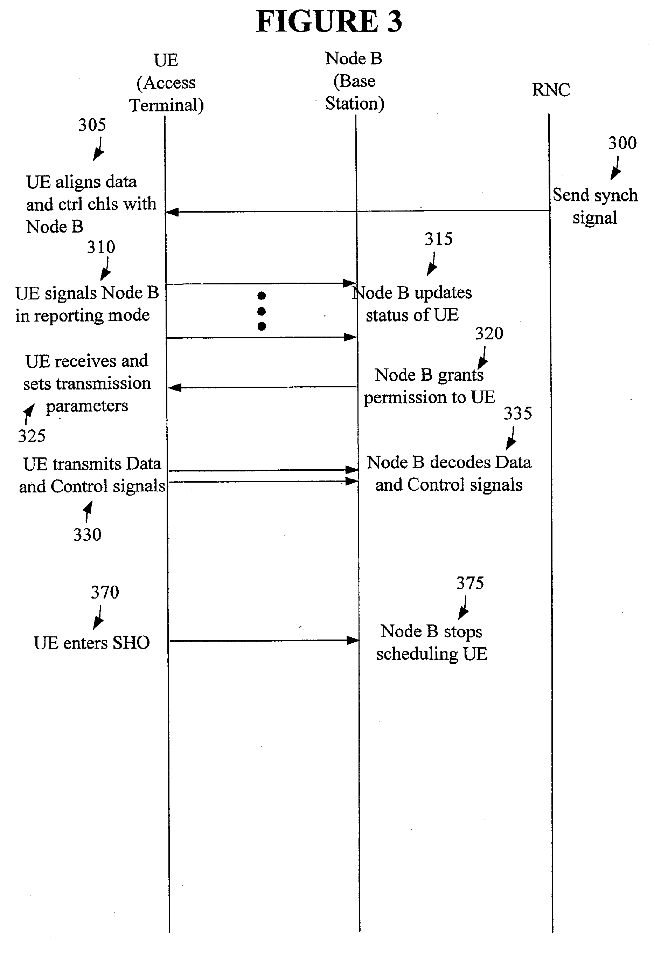

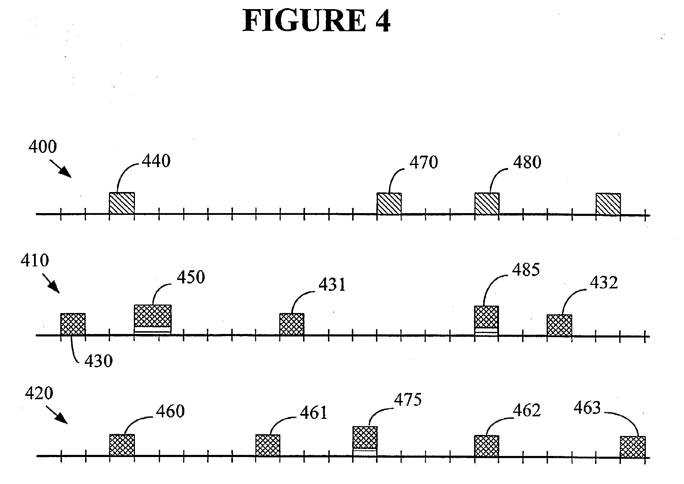

Normally, the channels 260, 270, 280 in the UE 120 communicate with the corresponding channels 220, 230, 240 in the Node B 130. Under the operation of the controllers 210, 250 the channels 220, 260; 230, 270; 240, 280 are used to effect a controlled time scheduling for communications from the UE 120 to the Node B 130. For example, the control channel 280 is generally used by the UE 120 to request permission to transmit data and/or control information to the Node B 130. The shared channel 220 is used by the Node B 130 to notify the UE 120 of the circumstances under which it may transmit to the Node B 130 via the data and control channels 270, 280. The process for effecting such a communication session is described in greater detail in conjunction with the flow diagram of Figure 3 and the timing diagram of Figure 4.

Generally, the UE has a first and second status in which it may operate in the network. In the first status, the UE 120 is in contact with a plurality of Node Bs 130, which is sometimes referred to as a soft handoff ("SHO"). In the second status, the UE 120 is in contact with only one of the Node Bs 130, which is called the serving Node B. The methodology described herein is a method to coordinate UE transmissions on the uplink when UEs are not in SHO. The method is referred to as "time scheduled" mode of operation. The following description and drawings are presented with reference to the UE 120 entering and leaving the SHO status, and being in the time scheduled mode of operation.

Turning now to Figure 3, a flow diagram illustrating the interoperation of one of the Node Bs 130 and one of the UEs 120 of Figures 1 and 2 is shown. In the flow diagram of Figure 3, the exemplary UE 120 has entered the scheduled mode of operation, and thus, the RNC 138 provides a signal (at 300) through the Node B 130 to align or synchronize the operation of the Node B 130 and the UE 120. In the illustrated embodiment, different Node Bs 130 that are assigned to different RNCs 138 are operating with different system clocks from the instant RNC 138 and Node B 130. Therefore, to insure proper timing of signals between the Node B 130 and the UE 120, the first operation is to synchronize at least the communications between the two devices. In the illustrated embodiment, the UE 120 (at 305) responds to the synchronization signal by aligning (or realigning) the timing of signals to be delivered over the control and data channels 280, 270. In alternative systems that utilize a universal clock signal, synchronization may not be necessary when the UE 120 first enters the scheduled mode of operation with the Node B 130, as it may already be synchronized.

The UE 120 has voice and/or data signals that it desires to transmit to the Node B. However, before the UE 120 is allowed to transmit data/voice to the Node B 130, it must first request, and be granted, permission. Accordingly, the UE 120 (at 310) periodically sends a reporting signal over the control channel 280 indicating that it has data/voice to be transmitted to the Node B 130. The Node B 130 (at 315) receives the signal on its control channel 240, updates the status of the UE 120, indicating that the UE 120 is in the scheduled mode of operation and desires to transmit data/voice information. The Node B 130 also determines certain information from parameters, such as quality and strength, of the signal received from the UE 120. For example, based on the quality and strength of the signal, the Node B 130 may determine that the UE 120 needs to adjust its transmitting power (increase or decrease).

The Node B 130 (at 320) responds to the request from the UE 120 by delivering a signal over the shared channel 220, granting permission to the Node B 130 to deliver its voice/data signal over the data channel 270. The Node B 130 grants permission to a selected on of the UEs 120 based on information obtained on the control channel 240 from the UE about the available power each UE 120 has for transmission and the data/voice buffer size for each UE 120. The permission granting signal delivered by the Node B 130 may include additional information, such as the time at which the UE 120 is permitted to transmit its voice/data and the period in which it must complete, or otherwise cease, the transmission. In the illustrated embodiment, the Node B 130 grants permission to the UE 120 to provide its data/voice signal, such as a packet of data, during a subsequent clock cycle, such as the next clock cycle in the illustrated embodiment. Depending on the load experienced by the Node B 130, it may not be able to respond immediately to the UE 120, and in fact, the UE 120 may not be granted permission to transmit the data/voice information until after two or more of the periodic reporting signals are generated.

Additionally, the Node B 130 may also provide information regarding the parameters of the data/voice signal to be provided by the UE 120. For example, the Node B 130, using information derived from a prior signal received from the UE 120 may indicate that the UE 120 should adjust the power and/or timing of its signal. Generally, the Node B 130 may request that the UE 120 adjust the parameters of its signal so as to conserve power within the UE 120, improve the quality of the signal received from the UE 120, and the like. For example, increasing the power of a weak signal may improve the quality of the transmission. Alternatively, decreasing the power of an overly strong signal may conserve power with the UE 120 without a corresponding loss of quality in the transmitted signal. Using information derived from a prior signal received from the UE 120, the Node B 130 can also derive information about the data rate, transmission duration, modulations and coding rate that a UE 120 should use when it starts the uplink data transmission. Thus, the Node B 130 may provide some, all or none of this information to the UE 120 in the signals delivered over the shared channel 220.

The UE 120 (at 325) examines the signal from the Node B 130 and determines which, if any, of its transmitting parameters need to be adjusted. In some circumstances, the UE 120 may heed any and all requests to modify its transmission. In other instances, the UE 120 may elect to override a request from the Node B 130, and transmit its signal using different parameters. For example, a UE 120 may be rated to transmit at a certain preselected data rate that requires more power than it is currently available to the UE 120. In this case, the UE 120 may decided to transmit with a lower data rate than is granted by the Node B 130. The UE 120, in this case, is responsible of informing the Node B 130 of the new transmitting parameters through the control channel 280, 240. Similarly, for example, if the UE 120 is granted permission to transmit a large amount of data, but then determines that it does not have that much data to transmit, then the UE 120 can change the transmit parameters in order to transmit its data. For example, the UE 120 may reduce the transmission time interval, or the UE 120 may reduce the modulation size, or the UE 120 may decrease the coding rate when transmitting the data. In this case, UE 120 is again responsible for notifying the serving Node B 130 of any changes it made through the control channel 280, 240.

At the appointed time, the UE 120 (at 330) begins transmitting its voice/data signal or packet over the data channel 270. At substantially the same time, the UE 120 may also transmit a signal over the control channel 280. The UE 120 may use the control channel 280 to indicate parameters associated with the signals being provided over the data channel 270. For example, at least in those instances where the UE 120 has overridden the parameters suggested by the Node B 130, it is useful for the UE 120 to provide information regarding the parameters that it used in transmitting the voice/data signals on the data channel 270. thus, the Node B 130 may use the information provided on the control channel 280 to decode information received on the data channel 230.

The Node B 130 (at 335) receives the information provided over both the data and control channels 230, 240. The information on the control channel 240 is decoded and used to decode the voice/data signal provided over the data channel 230. The decoded voice/data signals are then forwarded through the interface unit 200 to the various components of the system 100.

Ultimately, the UE 120 may travel to a new location where it is in communication with more than one of the Node Bs 130. At this point, the UE 120 will leave the scheduled status and enter the soft handoff status where the scheduled status is not supported. This change in status is communicated to the Node B 130 (at 370) by the UE 120 delivering a preselected signal over the control channel 270. The Node B (at 375) responds to the signal that the UE 120 is no longer in the scheduled mode of operation by discontinuing any scheduling of the UE 120. Thus, if the UE requests permission to transmit data, but then leaves the scheduling mode prior to the Node B 130 granting permission, then the UE 120 provides a signal over its control channel 280 indicating that it is no longer in the scheduling mode of operation. Thus, the Node B 130 will not send a signal granting permission to the UE 120 to transmit the data, but rather, the previous requests submitted by the UE is ignored.

Turning now to Figure 4, a timing diagram illustrating scheduling and transmission of data between a Node B 130 and a pair of UEs 120 is illustrated. The top line 400 of timing information illustrates the timing of signals delivered by the Node B 130 on the shared channel 220. The middle line 410 of timing information illustrates the timing of signals delivered by a first one of the UEs 120 on the data and control channels 270, 280. The bottom line 420 of timing information illustrates the timing of signals delivered by a second one of the UEs 120 on the data and control channels 270, 280. The top, bottom and middle lines 400, 410, 420 are related by their use of the same relative time scale.

The first one of the UEs 120 indicates that it has voice/data ready to transmit by entering into the reporting mode of operation and periodically generating a signal 430, 431, 432 on its control channel 280. In the illustrated embodiment, the Node B 130 responds with a signal 440 on its shared channel 220 granting permission to the first one of the UEs 120. The signal transmitted by the Node B 130 also includes information regarding whether the first one of the UEs 120 is being granted permission to transmit new data or to retransmit a previous packet of data. Under some circumstances, the Node B 130 may fail to properly receive a packet of data transmitted by the UE 120. Thus, provision is made to allow the Node B 130 to request that the packet of data be resent. In the illustrated embodiment, the Node B 130 has indicated that the first one of the UEs 120 is being granted permission to send a new packet of data. Accordingly, the first one of the UEs 120 responds to the signals by transmitting new data 450. In the illustrated exemplary embodiment, the transmission time interval for a data packet can be many time slots (TSs). Each interval between adjacent tick marks in Figure 4 is a fixed number of time slots. In UMTS, each time slot is 0.67 msec, and there are 3 TSs between adjacent tick marks. Those skilled in the art, however, will appreciate that the interval between adjacent tick marks may be any integer number. In the illustrated embodiment, the packet of data 450 is shown being transmitted over 5 TSs slots which is 1 and 2/3 of the tick mark interval. The UE 120 "knows" how long it can transmit since the transmission time interval is signaled from the Node B 130 using the shared channel 220. Generally, the UE 120 will transmit for as long a period of time as the Node B tells it to.

As seen on the time line 420, the second one of the UEs 120 has also indicated that it has voice/data ready to transmit by entering into the reporting mode of operation and periodically generating a signal 460, 461, 462, 463 on its control channel 280. Permission, however, is not granted by the Node B 130 immediately. In fact, the second one of the UEs 120 generates two periodic signals 460, 461 and the first one of the Node Bs 120 generates a single periodic signal 431 before the Node B responds. The Node B 130 responds with a signal 470 on its shared channel 220 granting permission to the second one of the UEs 120 to transmit new data. Accordingly, the second one of the UEs 120 responds to the signals by transmitting new data 475 during the next clock cycle.

It should be noted that the new data 475 is being transmitted during the clock cycle in which a periodic signal 460, 461, 462, 463 is scheduled to be sent. However, the UE 120 utilizes a prioritizing scheme in which the transmission of packets on the data channel 270 is assigned a higher weight than the transmission of the periodic signal. Accordingly, in those instances where the transmission of packets on the data channel 270 coincides with the transmission of the periodic signal on the control channel 280, the periodic signal is at least temporarily pre-empted. It should be remembered that the packets on the data channel 270 are accompanied by control signals on the control channel 280. Thus, there is a conflict between the control signals associated with the data packets and the periodic signal, which is resolved by at least temporarily suppressing the periodic signal, as shown in Figure 4.

The Node B 130 then responds to the reporting mode signal 431 of the first one of the UEs 120 by delivering a signal 480 granting permission to the first one of the UEs 120 to send its data/voice packet. The signal 480, however, implicitly indicates that the packet of data associated with the signal 450 was not received properly, as it requests that the packet be resent. Accordingly, the first one of the UEs 120 responds by resending (at 485) the packet of data and any associated control information on its data and control channels 270, 280.

Those skilled in the art will appreciate that the various system layers, routines, or modules illustrated in the various embodiments herein may be executable control units (such as the controllers 210, 250 (see Figure 2)). The controllers 210, 250 may include a microprocessor, a microcontroller, a digital signal processor, a processor card (including one or more microprocessors or controllers), or other control or computing devices. The storage devices referred to in this discussion may include one or more machine-readable storage media for storing data and instructions. The storage media may include different forms of memory including semiconductor memory devices such as dynamic or static random access memories (DRAMs or SRAMs), erasable and programmable read-only memories (EPROMs), electrically erasable and programmable read-only memories (EEPROMs) and flash memories; magnetic disks such as fixed, floppy, removable disks; other magnetic media including tape; and optical media such as compact disks (CDs) or digital video disks (DVDs). Instructions that make up the various software layers, routines, or modules in the various systems may be stored in respective storage devices. The instructions when executed by the controllers 210, 250 cause the corresponding system to perform programmed acts.

The particular embodiments disclosed above are illustrative only, as the invention may be modified and practiced in different but equivalent manners apparent to those skilled in the art having the benefit of the teachings herein. Furthermore, no limitations are intended to the details of construction or design herein shown, other than as described in the claims below. Consequently, the method, system and portions thereof and of the described method and system may be implemented in different locations, such as the wireless unit, the base station, a base station controller and/or mobile switching center. Moreover, processing circuitry required to implement and use the described system may be implemented in application specific integrated circuits, software-driven processing circuitry, firmware, programmable logic devices, hardware, discrete components or arrangements of the above components as would be understood by one of ordinary skill in the art with the benefit of this disclosure. It is therefore evident that the particular embodiments disclosed above may be altered or modified and all such variations are considered within the scope and spirit of the invention. Accordingly, the protection sought herein is as set forth in the claims below.

## Claims

1. A method for controlling a flow of information in a communications system, comprising:
transmitting a signal requesting to transmit information;
receiving a signal identifying a time at which information is permitted to be transmitted; and
transmitting at the identified time a signal containing the information.

2. A method, as set forth in claim 1, further comprising:
transmitting at the identified time a signal containing information usable to decode the information transmitted at the identified time.

3. A method, as set forth in claim 1, wherein transmitting the signal requesting to transmit information further comprises transmitting over a control channel a signal requesting to transmit information.

4. A method, as set forth in claim 1, wherein transmitting the signal requesting to transmit information further comprises periodically transmitting a signal requesting to transmit information.

5. A method, as set forth in claim 4, wherein periodically transmitting the signal requesting to transmit information further comprises temporarily suppressing periodically transmitting the signal requesting to transmit information in response to the periodic signal coinciding with transmitting at the identified time the signal containing the information.

6. A method, as set forth in claim 1, wherein transmitting at the identified time the signal containing the information further comprises transmitting at the identified time over a data channel the signal containing the information.

7. A method, as set forth in claim 1, wherein receiving the signal identifying the time at which information is permitted to be transmitted further comprises receiving over a shared channel the signal identifying the time at which information is permitted to be transmitted.

8. A method, as set forth in claim 1, wherein receiving the signal identifying the time at which information is permitted to be transmitted further comprises receiving a signal indicating whether new information is to be transmitted or old information is to be retransmitted.

9. A method for controlling the flow of information, comprising:
receiving a signal requesting to transmit information;
transmitting a signal identifying a time at which information is permitted to be transmitted; and
receiving at the identified time a signal containing the information.

10. A method as set forth in claim 9, wherein:
receiving the signal requesting to transmit information further comprises receiving over a control channel the signal requesting to transmit information;
transmitting the signal identifying the time at which information is permitted to be transmitted further comprises transmitting over a shared channel the signal identifying the time at which information is permitted to be transmitted; and
receiving at the identified time the signal containing the information further comprises receiving over a data channel at the identified time the signal containing the information.
